Europäisches Patentamt

(19) European Patent Office

Office européen des brevets

(11) Numéro de publication: **0 278 817**
A1

(12) # DEMANDE DE BREVET EUROPEEN

(21) Numéro de dépôt: 88400117.3

(22) Date de dépôt: **20.01.88**

(51) Int. Cl.⁴: **C 12 N 5/00**

(30) Priorité: **22.01.87 FR 8700741**

(43) Date de publication de la demande:
**17.08.88 Bulletin 88/33**

(84) Etats contractants désignés:
**AT BE CH DE ES FR GB GR IT LI LU NL SE**

(71) Demandeur: **SANOFI, société anonyme**
**40, Avenue George V**
**F-75008 Paris (FR)**

(72) Inventeur: **Lugassy, Claire**
**18, rue Laplace**
**F-75005 Paris (FR)**

**Escande, Jean Paul**
**15 rue Gambetta**
**F-92260 Fontenay aux Roses (FR)**

(74) Mandataire: **Gillard, Marie-Louise et al**
**Cabinet Beau de Loménie 55, Rue d'Amsterdam**
**F-75008 Paris (FR)**

(54) **Procédé de reconstitution in vitro de tissus.**

(57) La présente invention concerne un procédé de reconstitution in vitro de tissus.

Selon ce procédé, on soumet à une incubation en présence de collagène une suspension cellulaire préparée à partir de la population cellulaire hétérogène sortie de l'ensemble formé par une lattice et un explant d'un tissu pathologique mis en culture selon la méthode de culture sous lattice compacte couvrante, ladite population étant constituée, d'une part, de cellules pathologiques différenciées et, d'autre part, de fibroblastes des deux types, ceux provenant du stroma du tissu et ceux issus de la lattice.

EP 0 278 817 A1

# 0 278 817

**Description**

## Procédé de reconstitution in vitro de tissus

La présente invention a pour objet un procédé de reconstitution in vitro de tissus en trois dimensions.

Le screening des molécules pouvant servir de principe actif pour un médicament en vue du traitement d'un tissu pathologique est facilité si l'on dispose de cultures de cellules de ce tissu permettant une expérimentation in vitro. La méthode de première culture d'explants de tissus - maintenant connue sous l'appellation méthode de culture sous lattice compacte couvrante - décrite par Claire LUGASSY, Odile ENJOLRAS et Jean-Paul ESCANDE (C.R. Acad. Sc. Paris, t.301, Série III, no 17, pp. 779-784, 1985) s'est révélée d'une aide particulièrement efficace à cet effet.

Cette méthode de primo-culture d'explants de tissus permet d'obtenir avec un rendement élevé une rediffférenciation des fibroblastes issus des cellules mises en culture , les cellules ainsi rediffférenciées présentent les caractères habituels propres à leur type cellulaire et se multipliant de façon persistante pendant au moins plusieurs semaines. Elle consiste, avant incubation, à recouvrir les fragments de l'explant d'une matrice compacte formée d'un réseau de collagène contenant des fibroblastes vivants.

Cette méthode convient à la culture d'explants préparés à partir soit de tissus normaux, soit de tissus pathologiques et notamment dans ce cas de tissus tumoraux, qu'il s'agisse alors de tumeurs primitives ou de métastases. Elle est tout particulièrement adaptée à la première culture d'explants de mélanomes malins.

La préparation des explants se fait selon les techniques de l'art antérieur (C. AUBERT, Ann. Derm. Sypho., 102, (1), 1975 : 59-62). Les explants destinés à la mise en culture sont recueillis, éventuellement dissociés en fragments et placés sur le fond d'un récipient à fond plat constitué d'un matériau, tel qu'une matière plastique, sur lequel puissent adhérer les cellules.

La matrice compacte, également désignée ci-après par le terme lattice, est préparée selon la méthode décrite par E. BELL et coll. (Proc. Natl. Sc. U.S.A., 76 (1979, 1274-1276). On mélange du collagène vec des fibroblastes en présence d'un milieu de culture. Il se forme un gel qui se contracte progressivement sous l'action des fibroblastes, car ces cellules s'attachent aux fibres qui s'agencent alors en un réseau. Il en résulte une matrice au sein de laquelle les fibroblastes sont vivants.

Les fibroblastes utilisés pour la préparation de la lattice peuvent être de toutes origines. Les fibroblastes d'origine vasculaire sont particulièrement bien adaptés. De préférence, on utilise des fibroblastes préparés à partir d'un angiome. Les fibroblastes, après leur recueil à partir des tissus les contenant, peuvent être maintenus en culture par passages successifs jusqu'à leur utilisation.

Le collagène utilisé est obtenu à partir de tissus conjonctifs ; les tendons de la queue du rat sont une source facile d'emploi. On prépare une solution de collagène légèrement acide à l'aide par exemple d'acide acétique à 1 pour 1000 (v/v).

A la solution de collagène placée dans un récipient à fond plat tel qu'une boîte de Petri, sont ajoutés de préférence simultanément les fibroblastes, un milieu nutritif convenant au métabolisme de ces cellules ainsi qu'un agent neutralisant tel que de la soude.

Lorsqu'on utilise $5.10^5$ fibroblastes pour 2 ml d'une solution de collagène à 1 mg/ml coulés dans une boîte de Petri de 6 cm de diamètre, on obtient, en 3 à 8 jours, une lattice rétractée dont les dimensions vont de 5 à 10 mm de diamètre et 0,5 à 1 mm d'épaisseur.

Cette méthode de culture des explants consiste donc à recouvrir d'une lattice les explants adhérant préparés comme indiqué ci-dessus. Le milieu nutritif est ensuite versé de manière à baigner la lattice. Un mélange gazeux particulier convenant au métabolisme des cellules de l'explant et des fibroblastes, composé par exemple d'air additionné de 5 % de $CO_2$, peut être introduit. Le récipient ainsi préparé est mis en incubation à une température convenant au developpement des cellules qu'il renferme et de préférence à 37°C. Le milieu est par la suite régulièrement renouvelé, par exemple tous les cinq jours.

La demanderesse poursuivant ses travaux vient de montrer qu'il était maintenant possible de reconstituer des tissus en trois dimensions, à partir de cellules sortant de l'ensemble formé par une lattice et un explant d'un tissu pathologique mis en culture selon la méthode de culture sous lattice compacte couvrante décrite ci-dessus. Il s'agit là d'un résultat particulièrement important qui devrait permettre, par l'observation des tissus reconstitués et des populations cellulaires qui en sont issues, notamment en précisant les potentialités invasives des cellules, d'orienter les études cliniques et de parfaire le choix des médicaments appropriés au traitement du tissu pathologique concerné.

La présente invention concerne précisément, selon un premier aspect, un procédé de reconstitution in vitro d'un tissu en trois dimensions, qui consiste à soumettre à une incubation en présence de collagène une suspension cellulaire préparée à partir de la population cellulaire hétérogène sortie de l'ensemble formé par une lattice et un explant d'un tissu pathologique mis en culture selon la méthode de culture sous lattice compacte couvrante.

La population cellulaire hétérogène soumise à incubation selon l'invention comprend des cellules différenciées pathologiques et des fibroblastes de deux types, ceux provenant du stroma du tissu et ceux issus de la lattice.

L'explant mis en oeuvre est issu d'un tissu pathologique.Ce tissu peut être notamment une tumeur. Il peut alors s'agir d'une tumeur bénigne ou maligne. Le procédé selon l'invention convient particulièrement à la reconstitution de mélanomes malins.

2

Partant d'un tissu pathologique, la préparation des explants et leur première culture se font selon le mode opératoire décrit ci-dessus.

La mise en culture d'un explant recouvert d'une lattice conduit à la sortie de cellules quittant l'ensemble formé par l'explant et la lattice vers le milieu de culture et se fixant sur la paroi du flacon. Cette sortie est également observée lorsque l'ensemble formé par la lattice et l'explant est transféré du récipient où l'on a réalisé la première culture dans un autre récipient contenant un milieu nutritif neuf puis remis en incubation. Cette même sortie de cellules est encore observée chaque fois que l'on retransfère ledit ensemble dans un récipient contenant un milieu nutritif neuf.

Les cellules sortantes, quel que soit le moment où elles quittent l'ensemble formé par la lattice et l'explant soit à l'issue de la première culture de l'explant, soit après un transfert dudit ensemble, constituent une population hétérogène P1 comprenant, d'une part, des cellules différenciées et, d'autre part, des cellules d'aspect fibroblastique, les premières s'organisant en colonies reposant sur un tapis constitué par les secondes. L'addition d'un agent, tel que la trypsine, dans le flacon de culture permet de détacher les cellules qui adhèrent à la paroi du flacon. La population P1 peut ensuite être recueillie et mise en suspension dans un milieu nutritif convenant au métabolisme des cellules la constituant. Un milieu ayant les caractéristiques du milieu RPMI éventuellement complété par de la L-glutamine, du sérum de veau foetal et un ou plusieurs antibiotiques peut être notamment utilisé. Après avoir procédé à un dénombrement des cellules à l'aide, par exemple, d'une chambre de Thoma, on peut éventuellement ajuster le nombre de cellules. Une suspension contenant $2.10^6$ cellules par ml peut, par exemple, être préparée. La suspension ainsi obtenue est la suspension S1.

Le collagène mis en oeuvre dans le procédé de l'invention est obtenu à partir de tissus conjonctifs ; les tendons de la queue du rat sont une source facile d'emploi. On prépare une solution de collagène légèrement acide, le collagène étant après extraction et purification en solution par exemple dans une solution à 1 pour 1 000 (v/v) d'acide acétique. La solution de collagène mise en oeuvre peut contenir de 0,1 à 5 mg de collagène par ml. D'une manière préférée, elle contient 1 mg par ml.

A la solution de collagène placée dans un récipient à fond plat, tel qu'une boîte de Petri, sont ajoutés, de préférence simultanément, la suspension S1, du milieu nutritif ainsi qu'un agent neutralisant, tel que de la soude.

Lorsqu'on utilise une suspension S1 de $2.10^6$ cellules par ml sous un volume de 0,5 ml, pour 1,5 ml d'une solution de collagène à 1 mg par ml dans une boîte de Petri de 6 cm de diamètre, et en présence d'un milieu de culture, tel que le milieu RPMI, de 0,25 ml de NaOH 0,1M et de 0,45 ml de sérum de veau foetal, on obtient ainsi après 4 à 8 jours de rétraction un disque irrégulier de 5 à 10 mm de diamètre sur 1 à 2 mm d'épaisseur qui est un tissu reconstitué en trois dimensions. Ce tissu (tissu TR1) préparé à partir de la suspension cellulaire S1 est dit de première génération. Il est possible à partir de ce tissu TR1 de préparer en série d'autres tissus reconstitués.

Le tissu TR1 mis en culture donne issue par migration vers le milieu de culture à une population cellulaire P2 constituée également de cellules pathologiques différenciées et de cellules d'aspect fibroblastique. Cette population P2 mise en incubation en présence de collagène peut à son tour être utilisée pour fabriquer un ou plusieurs tissu(s) TR2 dit(s) de deuxième génération. Un tissu TR2 mis en culture donne alors issue à une population cellulaire P3 qui peut également à son tour être utilisée pour fabriquer un ou plusieurs tissu(s) TR3 dit(s) de troisième génération. On peut ainsi en répétant les mêmes opérations préparer une série de tissus reconstitués en trois dimensions.

Il convient de noter que, avant l'addition de trypsine ou d'un réactif équivalent dans le flacon contenant un tissu TR de énième génération et la population cellulaire n + 1 à laquelle il a donné issue, il est possible à l'aide, par exemple, d'une pipette de prélever le tissu TR et de le porter dans un second flacon contenant du milieu nutritif. Le tissu TR ainsi transféré peut alors donner issue à une seconde population cellulaire n + 1. Chacune des populations n + 1 peut être utilisée pour préparer un tissu TR de génération n + 1.

La présente invention concerne ainsi selon un autre aspect un procédé de préparation en série de tissus reconstitués, caractérisé par les opérations successives suivantes :

1. incubation en présence de collagène d'une suspension cellulaire préparée à partir de la population cellulaire hétérogène constituée de cellules pathologiques différenciées et de cellules d'aspect fibroblastique issues d'un tissu de première génération reconstitué selon le procédé de l'invention décrit ci-dessus, jusqu'à l'obtention d'un tissu reconstitué de deuxième génération,

2. incubation en présence de collagène d'une suspension cellulaire préparée à partir de la population cellulaire hétérogène constituée de cellules pathologiques différenciées et de cellules d'aspect fibroblastique issues du tissu reconstitué de deuxième génération obtenue en 1, jusqu'à l'obtention d'un tissu reconstitué de troisième génération.

Bien que généralement l'obtention d'un tissu reconstitué de quatrième génération puisse s'avérer plus difficile, la présente invention concerne également tout procédé de préparation en série de tissus reconstitués qui comprendrait en outre la répétition de l'opération 2, de telle sorte que, partant d'un tissu reconstitué de génération n, on obtienne un tissu reconstitué de génération n + 1.

L'invention est maintenant décrite en détail dans l'exemple illustratif ci-après.

EXEMPLE

PREPARATION EN SERIE DE MELANOMES MALINS RECONSTITUES EN TROIS DIMENSIONS

1. MATERIEL

- Tumeurs

Les explants tumoraux utilisés provenaient de quatre métastases cutanées (MCI à MCIV) de mélanomes malins prélevés chez quatre patients différents. Ils ont été cultivés par la méthode de culture sous lattice compacte couvrante.

- Collagène

Du collagène de type I a été extrait de queues de rats et préparé selon la technique connue (dont on trouvera une description dans Proc. Nat. Acad. Sci. USA, Vol. 76, n° 8, 1979, p. 1274), sous la forme d'une solution purifiée aqueuse à 1 pour 1 000 (v/v) d'acide acétique contenant 1 mg de collagène par ml.

- Milieu de culture complet

On a utilisé du milieu RPMI 1640, commercialisé par la Société GIBCO, auquel ont été ajoutés : 1 % de L-glutamine, 15 % (v/v) de sérum de veau foetal (S.V.F.) et, par millilitre, 100 unités de pénicilline et 100 mg de streptomycine.

- Flacon de culture

Des flacons plats dont le fond présente une surface de 25 cm$^2$ commercialisés par la Société Falcon ont été utilisés.

2. MODE OPERATOIRE

1 Préparation de mélanomes malins reconstitués de première génération (MMR1)

A - Préparation de la population de cellules $P_1$

Une solution de collagène dans l'acide acétique à 1 pour 1 000 (v/v) contenant 1 mg/ml de collagène est préparée à partir des tendons de la queue du rat.

Des fibroblastes sont obtenus à partir d'un angiome veineux humain évolutif de l'adulte. Ils sont maintenus en culture par passage successifs et sont utilisés entre le 9e et le 24e passage.

Les lattices sont préparées en ajoutant, pour chacune d'elles. à 2 ml de la solution du collagène coulés dans une boîte de Petri de 6 cm de diamètre et simultanément :

- 2 ml de milieu RPMI 1640 (G.E. MOORE et al ., J. Am. Med. Assoc.199, 8 (1967) 87-92) préalablement concentré 1,73 fois,
- 0,25 ml de NaOH 0,1 N,
- 0,45 ml de sérum de veau foetal, et
- 0,50 ml d'une suspension de fibroblastes dans du milieu de McCoy 5a modifié (S. IWAKARA ET J. GRACE J. Med. 1164, 64, 2279) contenant 10$^6$ cellules par ml.

En 3 à 8 jours, on obtient des lattices rétractées dont les dimensions varient de 5 à 10 mm de diamètre et 0,5 mm à 1 mm d'épaisseur.

Deux à trois fragments de chaque tumeur sont introduits dans un flacon de culture du type Falcon de 25 cm$^2$, et recouverts par une lattice. 2 ml de milieu A sont versés dans chaque flacon, ledit milieu A ayant la composition suivante :

- milieu RPMI 1640 additionné de :
. 1% de L-glutamine,
. 15% (v/v en final) de sérum de veau foetal,
. amphotéricine B (Fungizone R) à raison de 2,5 ng par ml,
. pénicilline à raison de 100 unités par ml,
. streptomycine à raison de 100 µg par ml.

Un mélange gazeux A (air plus 5% $CO_2$) y est introduit et les flacons sont ensuite placés à l'étuve à 37°C. Après 24 h d'incubation pour chaque flacon, la quantité de milieu est complétée à 5 ml. Le milieu est par la suite changé tous les cinq jours. A chaque changement, on renouvelle également le mélange gazeux A. Chaque flacon est inspecté quotidiennement au microscope. La durée d'observation s'est étendue sur 15 semaines.

Autour de l'ensemble explant-lattice, il se forme un tapis de cellules d'aspect fibroblastique d'où émergent des colonies de cellules tumorales mélanocytaires. Cet ensemble de cellules constitue la population P1. Ces cellules sont obtenues soit en primo-culture,soit après transfert et remise en culture dans un flacon neuf de l'ensemble constitué par l'explant tumoral et la lattice.

B - Préparation d'une suspension cellulaire S1

On verse dans le flacon contenant la population P1 0,5 ml d'une solution de trypsine à 0,25 % (p/v). La trypsine agit en détachant de la paroi du flacon les cellules qui y adhèrent. Après un contact avec la trypsine de 10 minutes, interrompu par addition de 5 ml de milieu complet, les cellules sont recueillies dans le milieu de culture . La suspension cellulaire ainsi obtenue est ajustée de manière à contenir $2.10^6$ cellules par ml (suspension S1).

C- Préparation d'une lattice à partir de la suspension S1

On réalise une lattice dans une boîte de Petri de 6 cm de diamètre en faisant rétracter à 37°C le collagène par la suspension S1 dans les conditions suivantes :

- solution de collagène    1,5 ml
- milieu de culture complet    2 ml
- NaOH, 0,1M    0,250 ml
- sérum de veau foetal    0,450 ml
- suspension S1    0,50 ml

On met donc en incubation $10^6$ cellules en présence de 1,5 ml d'une solution de collagène 1 mg/ml

D - Obtention d'un mélanome malin reconstitué

Après 4 à 8 h de rétraction à 37°C, la lattice se transforme en une masse cellulaire qui est la reconstitution d'un mélanome malin et à partir de laquelle on peut notamment observer une sortie de cellules. Le mélanome malin reconstitué ainsi obtenu est dit de première génération (mélanome MMR1).

2 Préparation en série de mélanomes malins reconstitués

Le mélanome MMR1 obtenu en 1C est transféré dans un second flacon contenant du milieu de culture complet et l'on place ce flacon en incubation à 37°C.

Le MMR1 donne issue par migration hors de sa masse vers le milieu de culture à une population cellulaire P2, constituée également de cellules mélanocytaires différenciées et de cellules d'aspect fibroblastique. A partir de cette population P2, on prépare une suspension S2 contenant $2.10^6$ cellules par ml selon le mode opératoire indiqué ci-dessus en 1A.

Un mélanome malin de deuxième génération (MMR2) est alors reconstitué à partir de la suspension S2 en procédant comme indiqué ci-dessus en 1B puis en 1C.

D'une manière identique, à partir de la suspension S3 préparée à partir de la population P3 également constituée de cellules mélanocytaires différenciées et de cellules d'aspect fibroblastique à laquelle donne issue le mélanome MMR2, un mélanome malin de troisième génération (MMR3) est reconstitué.

3 Transfert des mélanomes malins reconstitués

Chaque MMR est mis en culture jusqu'à ce que les cellules auxquelles il donne issue dans le flacon parviennent à confluence du fait de leur prolifération. Le MMR est alors transféré et remis dans un flacon neuf contenant du milieu de culture complet. L'opération est renouvelée toutes les 4 à 8 semaines en fonction de la rapidité de la croissance cellulaire.

4 Méthodes d'observation des mélanomes malins reconstitués et des populations cellulaires

- Observation macroscopique

Lors de chaque transfert, on observe :
* la rétraction, l'adhérence et la pigmentation du MMR
* la couleur du culot centrifugé après recueil des cellules à confluence, qui permet d'estimer la pigmentation cellulaire

- Observation microscopique

Les cellules issues des MMR in vitro sont observées à l'aide d'un microscope inversé du type Olympus ®C.K. avec contraste de phase.

5 Traitement des mélanomes malins reconstitués en vue de leur étude histologique

Les fragments des MMR inclus en paraffine sont colorés par l'hématéine éosine safran.

3. RESULTATS

1 Mélanomes malins reconstitués obtenus :

9 MMR ont été obtenus
- 3 à partir de la métastase MC I (cas I), à savoir :
    1 MMR de première génération (MMR I 1)

1 MMR de deuxième génération (MMR I 2)
1 MMR de troisième génération (MMR I 3)

- 1 à partir de la métastase MC II (cas II), à savoir :
    1 MMR de première génération (MMR II 1)

- 2 à partir de la métastase MC III (cas III), à savoir :
    1 MMR de première génération (MMR III 1)
    1 MMR de deuxième génération (MMR III 2)

- 3 à partir de la métastase MC IV (cas IV), à savoir :
    1 MMR de première génération (MMR IV 1)
    1 MMR de deuxième génération (MMR IV 2)
    1 MMR de troisième génération (MMR IV 3)

2 Suivi macroscopique et microscopique du contenu des flacons (cf. tableau I)

A - MMR de première génération

- Observation macroscopique des MMR I

TABLEAU I

| | Rétraction | Adhérence | Pigmentation | Culot cellulaire | Colonies tumorales | Tapis fibroblastique |
|---|---|---|---|---|---|---|
| **MMR1** | | | | | | |
| - I 1 | +++ | +++ | ++ | brun clair | +++ | ++ puis s'amenuise |
| - II 1 | ++ | +++ | +++ | brun | ++ puis 0 | ++ puis souffrance |
| - III 1 | ++ | +++ | + | jaunâtre | +++ | ++ puis s'amenuise |
| - IV 1 | ++ | +++ | ++ | brun | +++ | ++ puis s'amenuise |
| **MMR2** | | | | | | |
| - I 2 | ++ | ± | + | jaunâtre | ++ | + |
| - III 2 | ± | ± | 0 | blanc | ++ | 0 |
| - IV 2 | + | + | + | brun clair | ++ | ± |
| **MMR3** | | | | | | |
| - I 3 | + | 0 | 0 | blanc | ++ | ± |
| - IV 3 | ± | ± | ± | brun très clair | + | 0 |

La lattice préparée à partir de la suspension S1 est un disque translucide de 6 cm de diamètre. Après 4 à 8 jours de rétraction, il devient un disque irrégulier et blanchâtre de 5 à 10 mm de diamètre sur 1 à 2 mm d'épaisseur. Puis la rétraction peut se poursuivre. Elle a fourni par exemple, pour le cas I, une tumeur de 3 mm de diamètre pour le MMR1. L'adhérence au flacon des MMR1 mis en culture est importante. Au cours du temps, une pigmentation est apparue progressivement. Elle est demeurée inhomogène et discrète pour les MMR I 1 et III 1; elle a atteint le brun foncé pour le MMR IV 1, le noir pour le MMR II 1.

Le culot cellulaire obtenu à partir de la population P2 était brun clair pour le MMR I 1, brun pour les MMR II 1 et III 1, jaunâtre pour le MMR IV 1.

- Observation microscopique des populations cellulaires P1 et P2.

La population P1, utilisée dans la préparation des MMR1, était constituée dans les quatre cas I à IV de colonies tumorales mélanocytaires différenciées visibles à l'oeil nu dans le flacon et associées à un tapis de cellules d'aspect fibroblastique.

Les cellules tumorales différenciées étaient de type fibroïde pour le cas I, en fuseau plus globuleux pour les cas II et IV, de type épithélioïde pour le cas III. Elles formaient de volumineuses colonies sans aucune inhibition de contact.

* dans les quatre cas I à IV, la primo-culture de chaque MMR1 a donné une population P2 de même type que la population P1,

* lors des transferts successifs pour les MMR I 1, III 1 et IV 1, on a pu constater, après respectivement une observation de 20 mois pour le cas I 1, de 10 mois pour le cas III 1 et de 6 mois pour le cas IV 1, que les colonies qui, dès le départ étaient moins volumineuses, ont persisté alors que s'amenuisait le tapis de cellules d'aspect fibroblastique,

* pour le MMR II 1, on a pu constater que les cellules sont progressivement toutes devenues d'aspect fibroblastique, avec croissance ralentie et signes de souffrance (après six mois). Seul un MMR1 de première génération a été réalisé dans ce cas.

B - MMR de deuxième génération

Ils ont été réalisés avec les populations P2 issues respectivevement des MMR I 1, III 1 et IV 1.

- Observation macroscopique des MMR2

* la rétraction était moins importante que pour les MMR1, en particulier pour le MMR III 2 où le disque est resté flasque et d'environ 2 cm de diamètre. De même, l'adhérence au flacon était diminuée, surtout pour les MMR I 2 et III 2 qui se décollaient facilement,

* la pigmentation est restée partielle, touchant surtout la périphérie et la surface supérieure libre des MMR, pour les MMR I 2 et IV 2, complètement nulle pour le MMR III 2,

* le culot cellulaire obtenu à partir de la population P3 était plus clair que le culot P2 et complètement blanc pour les cellules issues du MMR III 2.

- Observation microscopique des populations cellulaires P3

La population P3 issue de chaque MMR2 comportait des cellules tumorales se multipliant à partir du MMR, mais les colonies disséminées étaient dans l'ensemble moins volumineuses, les cellules semblant avoir acquis une certaine inhibition de contact.

Les cellules d'aspect fibroblastique étaient, après une observation de respectivement quatre mois pour le MMR I 2, deux mois pour le MMR III 2 et trois mois pour le MMR IV 2, encore plus rares, limitées au pourtour du MMR pour les MMR I 2 et IV 2, complètement absentes pour le MMR III 2.

C - MMR de troisième génération

Ils ont pu être réalisés avec les populations P3 issues respectivement des MMR I 2 et IV 2, mais non avec la population P3 issue du MMR III 2 qui ne rétractait pas du tout le collagène.

- Observation macroscopique des MMR3

Pour les MMR I 3 et IV 3, les phénomènes déjà observés se sont accentués : rétraction moins importante, adhérence au flacon diminuée, pigmentation faible pour le MMR IV 3 et nulle pour le MMR I 3.

- Observation microscopique des populations cellulaires P4

On observe toujours une multiplication de cellules tumorales à partir de chaque MMR3, mais les colonies tumorales sont dans l'ensemble moins importantes. Les culots obtenus à partir des populations P4 issues des MMR3 sont apparus encore plus clairs. Enfin, on a constaté, après une observation de respectivement trois mois pour le MMR I 3 et d'un mois pour le MMR IV 3, que le tapis fibroblastique se raréfiait pour le MMR I 3 et avait même disparu pour le MMR IV 3.

3 Etude histologique des MMR

La présence de cellules volumineuses, polyédriques, comportant un large noyau central et nucléolé, dont certaines étaient très pigmentées, était très évocatrice d'une prolifération mélanocytaire tumorale.

## 4. COMMENTAIRES

Les cellules issues de la culture sous lattice compacte couvrante d'explants de mélanomes malins (métastases cutanées) peuvent donc être utilisées pour reconstituer in vitro, une fois mélangées à du collagène, une tumeur en trois dimensions dont il est facile de suivre l'évolution. Les paramètres les plus intéressants à suivre sont : la rétraction, la pigmentation et l'adhérence. Leur étude permet de suivre in vitro les diverses étapes de formation de la tumeur mélanocytaire maligne.

Les cellules issues des MMR se caractérisent par la variabilité de leurs potentialités à se multiplier, à se pigmenter, à former des colonies disséminées dans le flacon de culture à distance des MMR.

Il est possible d'établir un lien entre les caractéristiques évolutives d'un MMR et les particularités des cellules qui en sont issues. Pour l'essentiel, plus la pigmentation du MMR devient intense, moins la multiplication cellulaire et la migration sous forme de colonies disséminées trouvent facilement à se réaliser.

D'autre part, l'importance en taille et en nombre des cellules des colonies devient moindre au fur et à mesure que se raréfie le tapis de cellules d'aspect fibroblastique qui leur était associé.

Ces phénomènes deviennent plus caractéristiques encore lorsque l'on étudie la reconstitution en série de MMR.

Il est également possible d'envisager des facteurs spécifiques et distincts conditionnant en particulier les potentialités de pigmentation, de multiplication et de migration cellulaires.

## Revendications

1. Procédé de reconstitution in vitro d'un tissu en trois dimensions, caractérisé en ce qu'on soumet à une incubation en présence de collagène une suspension cellulaire préparée à partir de la population cellulaire hétérogène sortie de l'ensemble formé par une lattice et un explant d'un tissu pathologique mis en culture selon la méthode de culture sous lattice compacte couvrante, ladite population étant constituée, d'une part, de cellules pathologiques différenciées et de fibroblastes de deux types, ceux comprenant du stroma du tissu et ceux provenant de la lattice.

2. Procédé de reconstitution in vitro d'un tissu en trois dimensions selon la revendication 1, caractérisé en ce que l'explant mis en oeuvre est issu d'une tumeur.

3. Procédé de reconstitution in vitro d'un tissu selon la revendication 2, caractérisé en ce que la tumeur dont est issu l'explant est une tumeur maligne.

4. Procédé de reconstitution in vitro d'un tissu selon la revendication 3, caractérisé en ce que la tumeur dont est issu l'explant est un mélanome malin.

5. Procédé de reconstitution in vitro d'un tissu selon l'une quelconque des revendications 1 à 4, caractérisé en ce que le collagène est utilisé sous forme d'une solution purifiée aqueuse contenant de 0,1 à 5 mg de collagène par ml.

6. Procédé de reconstitution in vitro d'un tissu selon la revendication 5, caractérisé en ce que le collagène est utilisé sous forme d'une solution contenant 1 mg de collagène par ml.

7. Procédé de reconstitution in vitro d'un tissu selon l'une quelconque des revendications 4 à 6, caractérisé en ce que $2.10^6$ cellules de la population hétérogène issue de la culture d'un mélanome malin sont mises en incubation en présence de 1,5 ml d'une solution de collagène à 1 mg/ml.

8. Procédé de préparation en série de tissus reconstitués en trois dimensions, caractérisé par les opérations successives suivantes :

A - incubation en présence de collagène d'une suspension cellulaire préparée à partir de la population cellulaire hétérogène, constituée de cellules pathologiques différenciées et de cellules d'aspect fibroblastique, issue d'un tissu reconstitué de première génération obtenu selon le procédé de l'une quelconque des revendications 1 à 7, jusqu'à l'obtention d'un tissu reconstitué de deuxième génération.

B - incubation en présence de collagène d'une suspension cellulaire préparée à partir de la population cellulaire hétérogène, constituée de cellules pathologiques différenciées et de cellules d'aspect fibroblastique, issue du tissu reconstitué de deuxième génération obtenu en A, jusqu'à l'obtention d'un tissu reconstitué de troisième génération.

9. Procédé de préparation en série de tissus reconstitués selon la revendication 8, caractérisé en outre par la répétition de l'opération B, de telle sorte que, partant d'un tissu reconstitué de génération n, on obtienne un tissu reconstitué de génération n + 1.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | BIOLOGICAL ABSTRACTS, vol. 73, 1982, résumé no. 69842, Philadelphie, P.A., US; D.C. BENNETT et al.: "Reconstitution of branching tubules from 2 cloned mammary cell types in culture", & DEV. BIOL. 87(1), 193-199, 1981 <br> * Résumé * <br> --- | 1 | C 12 N 5/00 |
| Y | EP-A-0 061 549 (KUREHA KAGAKU KOGYO KABUSHIKI KAISHA) <br> * Page 7, ligne 28 - page 9, ligne 9 * <br> --- | 1 | |
| A | WO-A-8 001 350 (MASSACHUSETTS INSTITUTE OF TECHNOLOGY) <br> ----- | | |

DOMAINES TECHNIQUES RECHERCHES (Int. Cl.4)

C 12 N

**Le présent rapport a été établi pour toutes les revendications**

| Lieu de la recherche | Date d'achèvement de la recherche | Examinateur |
|---|---|---|
| LA HAYE | 26-04-1988 | REMPP G.L.E. |

EPO FORM 1503 03.82 (P0402)